# EUROPÄISCHE PATENTSCHRIFT

(11) **EP 1 716 224 B1**
(45) Veröffentlichungstag und Bekanntmachung des Hinweises auf die Patenterteilung: **03.06.2009**
(21) Anmeldenummer: 04798063.6
(22) Anmeldetag: 24.11.2004
(51) Int. Cl.: C11D 3/37, C11D 17/00, A61K 8/02, A61K 8/81, A61Q 5/02, A61Q 19/10

(54) **MEHRKOMPONENTEN-THIN-TO-THICK-SYSTEM**
MULTICOMPONENT THIN-TO-THICK SYSTEM
SYSTEME MULTICOMPOSANT DE FAIBLE A FORTE VISCOSITE

(30) Priorität: 13.12.2003 DE 10358536; 04.03.2004 DE 102004010485
(43) Veröffentlichungstag der Anmeldung: 02.11.2006
(60) Teilanmeldung: 09154885.9
(73) Patentinhaber: Henkel AG & Co. KGaA, 40589 Düsseldorf (DE)
(72) Erfinder: BEDRUNKA, Danuta, 41541 Dormagen (DE); HLOUCHA, Matthias, 50677 Köln (DE); HOFMANN, Rainer, 50389 Wesseling (DE); HATTEMER, Erik, 40597 Düsseldorf (DE); RYBINSKI, Wolfgang von, 40593 Düsseldorf (DE); DAHLMANN, Doris, 40589 Düsseldorf (DE); FREDERIKSEN, Matthias, 4053 Basel (CH)
(86) Internationale Anmeldenummer: PCT/EP2004/013316
(87) Internationale Veröffentlichungsnummer: WO 2005/056743

(56) Entgegenhaltungen:
- EP-A- 0 875 557
- EP-A- 0 982 021
- DE-A1- 19 752 163
- US-A- 5 004 557
- US-A- 5 362 415
- US-A- 5 652 208
- ILIOPOULOS, WANG, AUDEBERT: LANGMUIR, Nr. 7, 1991, Seiten 617-619, XP008042857 in der Anmeldung erwähnt

## Beschreibung

Gruppen des Polyacrylats. Die Bindung liegt vorzugsweise in Form einer Ester-Bindung an Carbonsäure-Gruppen vor. Alternativ kann die Alkylgruppe jedoch etwa auch über eine Alkylen-Gruppe, insbesondere C₁₋₆-Alkylen-Gruppe, vorzugsweise Methylen-Gruppe, an das Polyacrylat-Ruckgrat gebunden sein.

Der Modifizierungsgrad gibt hierbei das Verhältnis zwischen Anzahl der Monomereinheiten des Polyacrylats und Anzahl der hydrophoben modifizierenden Alkylgruppen an bzw. insbesondere, im Falle der Bindung an funktionelle Gruppen und vorausgesetzt, dass alle Monomere funktionelle Gruppe umfassen, die Anzahl der durch hydrophobe Alkylgruppen modifizierten funktionellen Gruppen in Bezug auf die Gesamtzahl der funktionellen Gruppen.

Die hydrophobe Alkylgruppe kann linear oder verzweigt sein. Bei der hydrophoben Gruppe handelt es sich hierbei erfindungsgemäß vorzugsweise um eine Alkylgruppe mit einer Länge zwischen C₈ und C₅₀, insbesondere zwischen C₈ und C₂₆, besonders bevorzugt zwischen C₁₂ und C₂₂, vor allem zwischen C₁₆ und C₂₀, insbesondere mit einer Länge von C₁₈.

Es handelt sich bei dem hydrophob modifizierten Polyacrylat in einer bevorzugten Ausführungsform um ein hydrophob modifiziertes Polyacrylat, wobei die hydrophobe Modifzierung des Polyacrylats durch Alkylgruppen bewirkt wird, die an Carboxylgruppen des Polyacrylats, insbesondere durch Esterbindung oder durch Säureamidbindung, gebunden sind.

Insbesondere kann es sich bei dem hydrophob modifizierten Polyacrylat um ein Polyacrylat handeln, das Einheiten der Formel -CH₂-C(R¹)(W)- und Einheiten der Formel -CH₂-C(R²)([X]ₘ-[Y]ₙ-[B]ₒ-Z-H)- umfasst, wobei
R¹ und R² unabhängig voneinander für Wasserstoff oder Methyl stehen,
W für COOH steht,
X für C(O)O-, oder (C₁-C₆)-Alkylen, insbesondere Methylen, besonders bevorzugt für C(O)O-, steht,
Y für (C₁-C₄)-Oxyalkylen, insbesondere Oxyethylen und/oder Oxypropylen, steht, wobei eine gemischte oder blockartige Anordnung vorliegen kann,
B für CO steht,
Z für (C₅-C₅₀)-Alkylen, vorzugsweise (C₅-C₃₅)-Alkylen, steht,
m für 0 oder 1, bevorzugt für 1, steht,
n einen Wert von 0 bis 50, vorzugsweise von 0 bis 30, besitzt,
o 0 oder 1 beträgt.

Alle der zuvor genannten Kohlenwasserstoffreste sowie unabhängig davon das Rückgrat des Polymers können gegebenenfalls auch ein- oder mehrfach substituiert sein, insbesondere durch Reste ausgewählt aus Halogen, insbsondere Fluor, Chlor oder Brom, Hydroxy, Alkoxy, insbesondere C₁₋₆-Alkoxy, Amino, Alkylamino, insbesondere C₁₋₆-Alkylamino, Aryl, insbesondere C₆₋₁₀-Aryl, Arylalkyl, insbesondere C₆₋₁₀-Aryl-C₁₋₆-alkyl, Carboxy, Carboxyester, insbesondere Carboxy-C₁₋₆-alkyl-ester, und cycloaliphatischen Resten.

Das durch Alkylgruppen hydrophob modifizierte Polyacrylat besitzt vorzugsweise ein Molekulargewicht kleiner als 100000 kDa, besonders bevorzugt zwischen 10 und 1000 kDa, insbesondere zwischen 50 und 600 kDa.

Der Modifizierungsgrad des hydrophob modifizierten Polyacrylats liegt vorzugsweise zwischen 1 und 10 %, besonders bevorzugt zwischen 2 und 5 %, vor allem zwischen 2,5 und 3,5 %.

Als Beispiele für erfindungsgemäß verwendbare hydrophob modifizierte Polyacrylate seien genannt von Rohm und Haas Acusol 801 S, Acusol 820, Aculyn 22 (Acrylates/Steareth-20 Methacrylate Copolymer) und Aculyn 28 (AcrylateslBeheneth-25 Methacrylate Copolymer), von Noveon Pemulen TR-1, Pemulen TR-2, Carbopol ETD2020 und Carbopol Ultrez 20 Polymer (jeweils Acrylates/C10-30 Alkyl Acrylate Crosspolymer) und Carbopol Aqua SF-1, von National Starch Structure 3001 (Acrylates/Ceteth-20 Itaconate Copolymer) und Structure 2001 (Acrylates/Palmeth-26 Itaconate Copolymer), von 3V Sigma Synthalen W2000 (Acrylates/Palmeth-25 Acrylate Copolymer) und von Clariant Aristoflex HMB.

Es handelt sich bei dem hydrophob modifizierten Polyacrylat in einer besonders bevorzugten Ausführungsform um ein hydrophob modifiziertes Polyacrylat, wobei die hydrophobe Modifzierung des Polyacrylats durch Alkylgruppen bewirkt wird, die an Carboxylgruppen des Polyacrylats, insbesondere durch Esterbindung gebunden sind, und wobei es sich bei den Alkylgruppen um solche mit einer Länge zwischen C₈ und C₂₆, besonders bevorzugt zwischen C₁₂ und C₂₂, vor allem zwischen C₁₆ und C₂₀, insbesondere mit einer Länge von C₁₈, handelt.

In einer weiteren besonders bevorzugten Ausführungsform handelt es sich bei dem hydrophob modifizierten Polyacrylat um ein Polymer, das unter dem Handelsnamen Acusol 801 S oder Acusol 820 (Rohm und Haas) erhältlich ist.

### Tenside

Bei dem erfindungsgemäß einsetzbaren Alkylsulfat der zweiten wässrigen Flüssigkeit handelt es sich vorzugsweise um ein Alkali- und insbesondere Natriumsalz der Schwefelsäurehalbester der C₁₂-C₁₈-Feialkohole, besonders bevorzugt um Dodecylsulfat.

Bei dem erfindungsgemäß einsetzbaren Fettalkoholethoxylat der zweiten wässrigen Flüssigkeit handelt es sich vorzugsweise um ein Fettalkoholethoxylat mit einem mittleren Ethoxylierungsgrad von weniger als 20 EO, besonders bevorzugt um ein Fettalkoholethoxylat umfassend einen C₁₂-C₁₈-Fettalkohol und 6-7 Ethoxylat-Einheiten.

Bei dem erfindungsgemäß einsetzbaren Tensid der ersten wässrigen Flüssigkeit handelt es sich vorzugsweise um ein Tensid ausgewählt aus der Gruppe bestehend aus anionischen Tensiden, amphoteren Tensiden und nichtionischen Tensiden oder Mischungen davon. Kationische Tenside können als zusätzliche Komponente in geringen Mengen vorhanden sein.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end-oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet.

Weitere geeignete anionische Tenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, welche als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete anionische Tenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Die anionischen Tenside können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Als nichtionische Tenside können beispielsweise alkoxylierte, vorteilhafterweise ethoxylierte, insbesondere primäre Alkohole mit vorzugsweise 8 bis 18 C-Atomen und durchschnittlich 1 bis 20, insbesodere 1 bis 12, Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Auch schwachschäumende nichtionische Tenside können eingesetzt werden, welche alternierende Ethylenoxid- und Alkylenoxideinheiten aufweisen. Unter diesen sind wiederum Tenside mit EO-AO-EO-AO-Blöcken bevorzugt, wobei jeweils eine bis zehn EO- bzw. AO-Gruppen aneinander gebunden sind, bevor ein Block aus den jeweils anderen Gruppen folgt. Beispiele hierfür sind Tenside der allgemeinen Formel 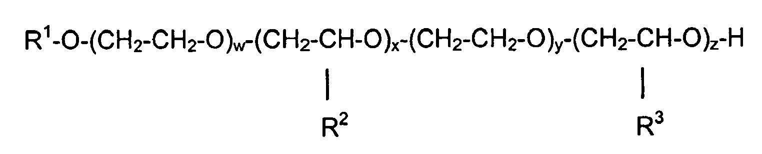 in der R¹ für einen geradkettigen oder verzweigten, gesättigten oder ein- bzw. mehrfach ungesättigten C₆₋₂₄-Alkyl- oder -Alkenylrest steht; jede Gruppe R² bzw. R³ unabhängig voneinander ausgewählt ist aus -CH₃; -CH₂CH₃, -CH₂CH₂-CH₃, CH(CH₃)₂ und die Indizes w, x, y, z unabhängig voneinander für ganze Zahlen von 1 bis 6 stehen. Diese lassen sich durch bekannte Methoden aus den entsprechenden Alkoholen R¹-OH und Ethylen- bzw. Alkylenoxid herstellen. Der Rest R¹ in der vorstehenden Formel kann je nach Herkunft des Alkohols variieren. Werden native Quellen genutzt, weist der Rest R¹ eine gerade Anzahl von Kohlenstoffatomen auf und ist in der Regel unverzeigt, wobei die linearen Resten aus Alkoholen nativen Ursprungs mit 12 bis 18 C-Atomen, z.B. aus Kokos-, Palm-, Talgfett- oder Oleylalkohol, bevorzugt sind. Aus synthetischen Quellen zugängliche Alkohole sind beispielsweise die Guerbetalkohole oder in 2-Stellung methylverzweigte bzw. lineare und methylverzweigte Reste im Gemisch, so wie sie üblicherweise in Oxoalkoholresten vorliegen. Unanbhängig von der Art des zur Herstellung der erfindungsgemäß in den Mitteln enthaltenen nicht-ionischen Tensiden eingesetzten Alkohols sind erfindungsgemäße Mittel bevorzugt, bei denen R¹ in der vorstehenden Formel für einen Alkylrest mit 6 bis 24, vorzugsweise 8 bis 20, besonders bevorzugt 9 bis 15 und insbesondere 9 bis 11 Kohlenstoffatomen steht. Als Alkylenoxideinheit, die alternierend zur Ethylenoxideinheit in den Niotensiden enthalten sein kann, kommt neben Propylenoxid insbesondere Butylenoxid in Betracht. Aber auch weitere Alkylenoxide, bei denen R² bzw. R³ unabhängig voneinander ausgewählt sind aus -CH₂CH₂-CH₃ bzw. CH(CH₃)₂, sind geeignet.

Eine weitere Klasse einsetzbarer nichtionischer Tenside, die entweder als alleiniges nichtionisches Tensid oder in Kombination mit anderen nichtionischen Tensiden eingesetzt werden können, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die erfindungsgemäß eingesetzt werden können, sind die Alkylpolyglycoside (APG). Einsetzbare Alkypolyglycoside haben die allgemeine Formel R-O(-G)_{z}, in der R für einen linearen oder verzweigten, insbesondere in 2-Stellung methylverzweigten, gesättigten oder ungesättigten, aliphatischen Rest mit 8 bis 22, vorzugsweise 8 bis 18 C-Atomen steht, G für einen glykosidisch gebundenen Rest eines Monosaccharids steht und z ein Wert zwischen 1 und 10 bedeutet.

Eine für den technischen Maßstab bedeutsame Synthese zur Herstellung der APG besteht im wesentlichen in der säurekatalysierten Kondensation von Monosacchariden vom Typ der Aldosen (HO-G) mit langkettigen Alkoholen (ROH), die 8 bis 22, vorzugsweise 8 bis 18 C-Atome enthalten. Unter Wasseraustritt entstehen Alkylglycoside der Formel (I)

R-O(-G)_{z} (I)

wobei der Wert von z durch die Wahl der Reaktionsbedingungen in weiten Grenzen variiert werden kann. Erfindungsgemäß brauchbar sind Alkylglycoside der Formel I mit n = 1 bis 10; bevorzugt werden Verbindungen mit Werten für n zwischen 1 und 6, insbesondere 1 bis 2. In Produkten, bei denen n größer als 1 ist, stellt n naturgemäß einen statistischen Mittelwert dar.

Bei der Herstellung der Alkylglycoside kann man auch von Oligo- oder Polysacchariden ausgehen, die dann im Verlauf der säurekatalysierten Reaktion zunächst durch Hydrolyse und/oder Alkoholyse zu niederen Bruchstücken depolymerisiert werden ehe sich die Alkylglycoside der Formel I bilden. Auch Gemische verschiedener reduzierender Monosaccharide oder Polysaccharide, die verschiedene Monosaccharideinheiten enthalten, lassen sich als Ausgangsmaterialien verwenden, wobei, falls n größer als 1 ist, entsprechend gemischt zusammengesetzte Alkylglycosidmoleküle entstehen können.

Als Ausgangsmaterialien eignen sich vorzugsweise folgende Monosaccharide: Glucose, Mannose, Galaktose, Arabinose, Apiose, Lyxose, Gallose, Altrose, Idose, Ribose, Xylose und Talose sowie die aus diesen Monosacchariden zusammengesetzten Oligo- und Polysaccharide, beispielsweise Maltose, Lactose, Maltotriose, Hemicellulose, Stärke, Partialhydrolisate der Stärke und Zuckersirup. Im Rahmen der Erfindung werden allerdings Alkylglycoside bevorzugt, die aus gleichen Monosaccharideinheiten aufgebaut sind. Besonders bevorzugt werden dabei Alkylglycoside, bei denen der Rest (-G) von der Glucose abgeleitet ist. Für diese auch als Alkylglucoside bezeichneten Verbindungen werden entsprechend als Ausgangsmaterialien Glucose, Maltose, Stärke und andere Oligomere der Glucose verwendet.

Der Alkylteil R leitet sich bei der oben beschriebenen Herstellung von langkettigen, gegebenenfalls ungesättigten, vorzugsweise primären Alkoholen ab, die auch verzweigt sein können. Beispiele sind die synthetischen Oxoalkohole mit 9 bis 15 C-Atomen und die aus natürlichen Fettsäuren gewonnenen Fettalkohole mit 8 - 22 C-Atomen. Bevorzugt werden die Fettalkohole mit 8 bis 18 C-Atomen sowie die Oxoalkohole mit 11 bis 15 C-Atomen, insbesondere aber die Fettalkohole mit 8 bis 10 C-Atomen oder mit 12 bis 14 C-Atomen.

Eine weitere Klasse einsetzbarer nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete nichtionische Tenside sind Polyhydroxyfettsäureamide der Formel (II), 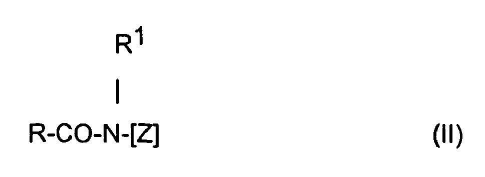 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (III), 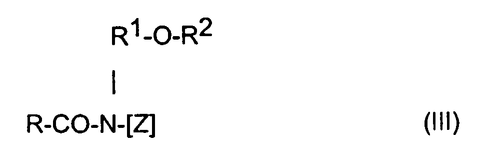 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder Propxylierte Derivate dieses Restes.

[Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können dann durch Umsetzung mit Fettsäuremethylestern in Gegenwart eines Alkoxids als Katalysator in die gewünschten Polyhydroxyfettsäureamide überführt werden.

Weitere einsetzbare nichtionische Tenside sind die endgruppenverschlossenen poly(oxyalkylierten) Tenside der Formel

R¹O[CH₂CH(R³)O]ₓ[CH₂]ₖCH(OH)[CH₂]ⱼOR²

in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, isoPropyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der obenstehenden Formel unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Geeignete amphotere bzw. zwitterionische Tenside, die erfindungsgemäß verwendet werden können, enthalten eine basische und eine saure Gruppe, die ein inneres Salz ausbilden. Bei der kationischen Gruppe handelt es sich hierbei insbesondere um eine quaternäre Ammoniumgruppe, es kann sich aber beispielsweise auch um eine Phosphonium-, Imidazolium- oder Sulfonium-Gruppe handeln. Bei der anionischen Gruppe handelt es sich insbesondere um eine Carboxylat- oder Sulfonat-Gruppe, es kann sich aber beispielsweise auch um eine Phosphonat- oder Sulfat-Gruppe handeln.

Bei erfindungsgemäß bevorzugt einsetzbaren amphoteren Tensiden handelt es sich um Verbindungen der allgemeinen Formel (IV) 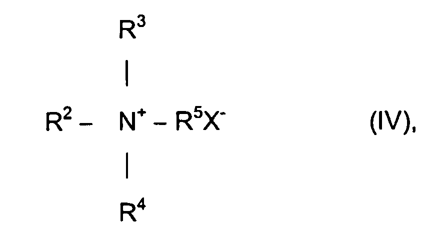 wobei R² vorzugsweise eine aliphatische oder aromatische hydrophobe, gegebenenfalls substituierte Gruppe ist, R³ und R⁴ unabhängig voneinander vorzugsweise Wasserstoff oder eine kurze, gegebenenfalls substituierte Alkylgruppe sind, und wobei R³ und R⁴ auch miteinander verknüpft sein können, und wobei R⁵ vorzugsweise eine gegebenenfalls substituierte Alkylen- oder Polyalkoxy-Gruppe ist, und wobei X⁻ vorzugsweise für eine Carboxylat- oder Sulfonat-Gruppe steht.

In einer bevorzugten Ausführungsform werden Betain-Verbindungen der Formel (V) eingesetzt, 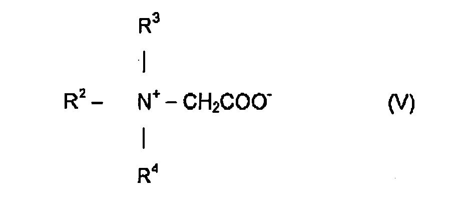 in der R² einen gegebenenfalls durch Heteroatome oder Heteroatomgruppen unterbrochenen Alkylrest mit 8 bis 25, vorzugsweise 10 bis 21 Kohlenstoffatomen und R³ und R⁴ gleichartige oder verschiedene Alkylreste mit 1 bis 3 Kohlenstoffatomen bedeuten. Bevorzugt sind C₁₀-C₁₈-Alkyldimethylcarboxymethylbetain und C₁₁-C₁₇-Alkylamidopropyldimethylcarboxymethyl-betain.

Ebenso bevorzugt sind Sulfobetaine. In diesem Fall ist statt der Carboxy- eine Sulfonatgruppe die anionische Gruppe. Besonders geeignet sind Bis(hydroxyethyl)sulfobetain und Cocoamidopropylsulfobetain. Es können jedoch allgemein Betaine und/oder Sulfobetaine eingesetzt werden, wie sie beispielsweise in US 2,082,275, US 2,702,279 oder US 2,255,082 beschrieben sind.

Besonders bevorzugt umfassen die eingesetzten Tenside der ersten wässrigen Flüssigkeit mindestens ein Tensid ausgewählt aus der Gruppe bestehend aus Alkylsulfaten, Alkylethersulfaten, Alkylsulfonaten, Alkylarylsulfonaten, Alkylbenzolsulfonaten, Alkylarylethersulfonaten, Alkyletheroxylaten, Fettalkoholethersulfaten, LAS, APG, Betainen und Fettalkoholethoxylaten, insbesondere aus der Gruppe Dodecylsulfat, Dodecylethersulfat und Fettalkoholethoxylate umfassend einen C₁₂-C₁₈-Fettalkohol und im Mittel weniger als 20 Ethoxylat-Einheiten, vorzugsweise 6-7 Ethoxylat-Einheiten.

Bei dem erfindungsgemäßen Doppelkamer-Systembeutel handelt es sich vorzugsweise um ein Doppelkammer-Spray oder eine Doppelkammer-Flasche. Eine manuelle Mischung der Flüssigkeiten ist hierbei nicht erforderlich, sondern die Mischung der Flüssigkeiten erfolgt automatisch bei Anwendung aufgrund der vorteilhaften Vorrichtung.

Die Applikation des Reinigungsmittels aus dem Behältnis, auf den Applikationsort kann hierbei insbesondere auch unter Verwendung eines Sprühspenders erfolgen.

Bevorzugt ist der Sprühspender hierbei ein manuell aktivierter Sprühspender, Insbesondere ausgewählt aus der Gruppe umfassend Aerosolsprühspender (Druckgasbehälter; auch u.a. als Spraydose bezeichnet), selbst Druck aufbauende Sprühspender, Pumpsprühspender und Triggersprühspender, insbesondere Pumpsprühspender und Triggersprühspender mit einem Behälter aus transparentem Polyethylen oder Polyethylenterephthalat. Sprühspender werden ausführlicher in der WO 96/04940 (Procter & Gamble) und den darin zu Sprühspendern zitierten US-Patenten. Triggersprühspender und Pumpzerstäuber besitzen gegenüber Druckgasbehältern den Vorteil, daß kein Treibmittel eingesetzt werden muß.

Hinsichtlich Beispielen für ein Doppelkammer-Behältnis wird auf die Anmeldungen WO 04/018319 und DE 102004007505.0 sowie auf den dort zitierten Stand der Technik verwiesen.

Zur Herstellung der hochviskosen Lösung werden die beiden Komponenten eines Zwei-Komponenten-Systems vorzugsweise in einem Verhältnis von 1:2 bis 100:1, besonders bevorzugt in einem Verhältnis von 1:1 bis 50:1 miteinander gemischt. Das bevorzugte Mischungsverhältnis ist hierbei natürlich abhängig von der Zusammensetzung der beiden Komponenten.

Die Zusammensetzungen der beiden Komponenten des Zwei-Komponenten-Systems und deren Mischungsverhältnis werden in einer bevorzugten Ausführungsform so gewählt, dass die Viskosität des Gemisches bei einer Scherrate von 0,1 s⁻¹ etwa doppelt so hoch, dreifach so hoch, fünffach so hoch oder zehnfach so hoch ist wie die Viskosität mindestens einer, vorzugsweise beider, der beiden Komponenten.

In einer weiteren bevorzugten Ausführungsform des erfindungsgemäßen Zwei-Komponenten-Systems werden die Zusammensetzungen und das Mischungsverhältnis der Komponenten so gewählt, dass die Viskosität der Mischung bei einer Scherrate von 0,1 s⁻¹ mehr als zehnfach, insbesondere mehr als 50fach, vor allem mehr als 100fach höher ist als die Viskosität mindestens einer, vorzugsweise beider der eingesetzten Komponenten.

In bevorzugten Ausführungsformen besitzen die Komponenten des Zweikomponentensystems unabhängig voneinander bei einer Scherrate von 0,1 s⁻¹ Viskositäten von kleiner als 100 und/oder kleiner als 200 Pas.

Die Viskositätsmessungen erfolgen mit Hilfe eines schubspannungskontrollierten Rotationsrheometers AR 1000-N (TA Instruments) mit einem Platte-Kegel-Meßsystem mit einem Durchmesser von 4 cm und einem Kegelwinkel von 1° in Fließversuchen im Schergeschwindigkeitsbereich von 0 bis 1000 s⁻¹ bei 20°C.

Der pH-Wert der Komponenten des Zweikomponenten-Systems und ebenso der des resultierenden Gemischs liegt vorzugsweise zwischen 5 und 14, wobei die einzelnen Komponenten des Zweikomponenten-Systems und/oder das resultierende Gemisch unterschiedliche pH-Werte haben können.

In einer bevorzugten Ausführungsform enthält mindestens eine der Komponenten zusätzlich mindestens einen weiteren Bestandteil, wobei die Auswahl des weiteren Bestandteils sich nach dem Verwendungszweck richtet.

Als Verwendungszweck des erfindungsgemäßen Zweikomponenten-Systems kommen beispielsweise die Verwendung als kosmetisches Mittel und/oder als Wasch- und/oder Reinigungsmittel in Frage.

Bei dem kosmetischen Mittel kann es sich hierbei insbesondere um ein Haarbehandlungs-und/oder Haarpflegemittel handeln, beispielsweise um ein Haarshampoo, eine Haarlotion, eine Haarkur oder ein Haarfärbemittel, oder um ein Körperpflegemittel, insbesondere Hautbehandlungsmittel, wie beispielsweise ein Deo, ein Sonnenöl oder eine Flüssigseife.

Bei dem Wasch- und/oder Reinigungsmittel kann es sich insbesondere um ein Textilbehandlungsmittel, insbesondere auch um ein Textilvorbehandlungsmittel, um ein Handwaschmittel, Handgeschirrspülmittel, insbesondere um ein Geschirrvorbehandlungsmittel, um ein Sanitärmittel, um ein Sterilisationsmittel und/oder um ein Desinfektionsmittel handeln.

Für die genannten Verwendungszwecke können entsprechend Inhaltsstoffe enthalten sein, wie sie dem Fachmann bekannt sind und/oder wie sie im folgenden für die Ausführungsformen Reiniger für harte Oberflächen, Haarpflegemittel und Vorbehandlungsmittel für Wäsche beispielhaft genannt werden.

### Reiniger für harte Oberflächen

Als harte Oberflächen im Sinne dieser Erfindung kommen insbesondere alle im Haushalt üblichen Flächen aus Kunststoff, Glas, Keramik oder Metall in Frage, beispielsweise Küchenoberflächen, Herde, Badezimmerflächen, Fußbodenfliesen, Laminatböden oder Geschirr.

Bei Verwendung des Zweikomponenten-Systems als Reiniger für harte Oberflächen können die Flüssigkeiten des Zweikomponenten-Systems auch weitere Bestandteile enthalten, wie sie für ein Reiniger von harten Oberflächen dem Fachmann bekannt sind. Neben dem hydrophob modifizierten Polymer und den zuvor genannten Tensiden kann es sich hierbei insbesondere um Gerüststoffe (Builder), Säuren, Alkalien, Hydrotrope, Lösungsmittel, Verdickungsmittel, Abrasivstoffe, Enzyme sowie weitere Hilfs- und Zusatzstoffe wie Konservierungsmittel, Farbstoffe, Duftstoffe (Parfüme), Korrosionsinhibitoren oder Hautpflegemittel handeln. Soll das Reinigungsmittel versprüht werden, kann gegebenenfalls auch ein Treibmittel enthalten sein. Die verschiedenen Flüssigkeiten des zweikomponentigen Reinigungsmittels können hierbei unabhängig voneinander eine oder mehrere der zuvor genannten und nachfolgend aufgezählten Komponenten in beliebiger Kombination und Konzentration enthalten.

Geeignete Builder sind beispielsweise Alkalimetallgluconate, -citrate, -nitrilotriacetate, -carbonate und -bicarbonate, insbesondere Natriumgluconat, -citrat und -nitrilotriacetat sowie Natrium- und Kaliumcarbonat und -bicarbonat, sowie Alkalimetall- und Erdalkalimetallhydroxide, insbesondere Natrium- und Kaliumhydroxid, Ammoniak und Amine, insbesondere Mono- und Triethanolamin, bzw. deren Mischungen. Hierzu zählen auch die Salze der Glutarsäure, Bernsteinsäure, Adipinsäure, Weinsäure und Benzolhexacarbonsäure sowie Phosphonate, und Phosphate. Die Mittel können Builder in Mengen, bezogen auf die Zusammensetzung, von 0,1 bis 5 Gew.-% enthalten.

Säuren und/ oder Alkalien dienen einerseits als pH-Regulatoren, andererseits können die Säuren aber auch zur Entfernung von Kalkflecken von den zu Säuren und/ oder Alkalien dienen einerseits als pH-Regulatoren, andererseits können die Säuren aber auch zur Entfernung von Kalkflecken von den zu reinigenden Oberflächen beitragen. Bei den erfindungsgemäß einsetzbaren Säuren kann es sich um anorganische Mineralsäuren, beispielsweise Salzsäure, und/oder um C₁₋₆- Mono-, Di-, Tri- oder Polycarbonsäuren oder - hydroxycarbonsäuren wie beispielsweise Ameisensäure, Essigsäure, Milchsäure, Citronensäure, Gluconsäure, Glutarsäure, Bernsteinsäure, Adipinsäure, Weinsäure oder auch Äpfelsäure sowie um weitere organische Säuren wie etwa Salicylsäure oder Amidosulfonsäure handeln. Besonders bevorzugt wird jedoch die Citronensäure eingesetzt; auch Mischungen aus mehreren Säuren können verwendet werden. Säuren können im erfindungsgemäßen Reinigungsmittel in Mengen von bis zu 6 Gew.-% enthalten sein, bezogen auf die Gesamtzusammensetzung.

Zu den gegebenenfalls einsetzbaren Basen zählen Alkanolamine, beispielsweise Mono- oder Diethanolamin, sowie Ammonium- oder Alkalimetallhydroxide, vor allem Natriumhydroxid. Das erfindungsgemäße Reinigungsmittel kann Basen in Mengen von bis zu 2,5 Gew.-% enthalten, bezogen auf die Gesamtzusammensetzung.

Die Komponenten des erfindungsgemäßen Mittels können weiterhin zur Viskositätsregulierung einen oder mehrere Verdicker enthalten. Als Verdickungsmittel geeignet sind dabei natürliche und synthetische Polymere sowie anorganische Verdickungsmittel. Zu den einsetzbaren Polymeren zählen Polysaccharide bzw. Heteropolysaccharide und andere organische natürliche Verdickungsmittel, darunter die Polysaccharidgummen wie Gummi arabicum, Agar, Alginate, Carrageene und ihre Salze, Guar, Guaran, Tragant, Gellan, Ramsan, Dextran oder Xanthan und ihre Derivate, z.B. propoxyliertes Guar, sowie ihre Mischungen, weiterhin auch Pektine, Polyosen, Johannisbrotbaumkernmehl, Stärke, Dextrine, Gelatine, Casein. Weiterhin können organische abgewandelte Naturstoffe wie Carboxymethylcellulose und -Celluloseether, Hydroxyethyl- und - propylcellulose und dgl. oder Celluloseacetat sowie Kernmehlether eingesetzt werden. Als organische vollsynthetische Verdickungsmittel können homo- und copolymere Polycarboxylate, vor allem Polyacryl- und Polymethacryl-Verbindungen sowie Vinylpolymere, Polycarbonsäuren, Polyether, Polyimine oder auch Polyamide dienen. Zu den einsetzbaren anorganischen Verdickungsmitteln zählen Polykieselsäuren, Tonmineralien wie Montmorillonite, Zeolithe, Kieselsäure sowie verschiedene nanopartikuläre anorganische Verbindungen wie nanopartikuläre Metalloxide, -oxidhydrate, -hydroxide, -carbonate und -phosphate sowie Silicate mit einer mittleren Teilchengröße von 1 bis 200 nm, bezogen auf den Teilchendurchmesser in der Längsrichtung, d.h. in der Richtung der größten Ausdehnung der Teilchen. Diese nanopartikulären Stoffen können optional in einer weiteren Ausführungsform der Erfindung mit einem oder mehreren Oberflächenmodifikationsmitteln behandelt sein. Die Oberflächenmodifikation erfolgt in dem Fachmann bekannter Weise mit ein- und mehrbasischen C₂₋₈-Carbonsäuren oder -Hydroxycarbonsäuren, funktionellen Silanen des Typs (OR)₄₋ₙSiRₙ (R = org. Reste mit funktionellen Gruppen wie Hydroxy, Carboxy, Ester, Amin, Epoxy, etc.), quartären Ammoniumverbindungen oder Aminosäuren sowie weiteren hierzu einsetzbaren Stoffen.

Neben den bisher genannten Verdickungsmitteln kann das erfindungsgemäße Reinigungsmittel Elektrolytsalze enthalten. Diese können ebenfalls zu einer Viskositätserhöhung beitragen. Elektrolytsalze im Sinne der vorliegenden Erfindung sind Salze, die in dem erfindungsgemäßen wäßrigen Mittel in ihre ionischen Bestandteile zerfallen. Bevorzugt sind die Salze, insbesondere Alkalimetall- und/oder Erdalkalimetallsalze, einer anorganischen Säure, vorzugsweise einer anorganischen Säure aus der Gruppe, umfassend die Halogenwasserstoffsäuren, Salpetersäure und Schwefelsäure, insbesondere die Chloride und Sulfate. Im Rahmen der erfindungsgemäßen Lehre kann ein Elektrolytsalz auch in Form seines korrespondierenden Säure/Base-Paares eingesetzt werden, beispielsweise Salzsäure und Natriumhydroxid anstatt Natriumchlorid.

In den Komponenten des erfindungsgemäßen Mittels sind die genannten oder andere organische und/oder anorganische Verdicker vorzugsweise nur in geringen Mengen enthalten. In einer bevorzugten Ausführungsform wird auf den Zusatz von Verdickern ganz verzichtet.

Die Komponenten des erfindungsgemäßen Mittels können vorteilhafterweise zusätzlich ein oder mehrere wasserlösliche organische Lösungsmittel enthalten, üblicherweise in einer Menge von bis zu 6 Gew.-%, bezogen auf die Gesamtzusammensetzung. Das Lösungsmittel wird im Rahmen der erfindungsgemäßen Lehre nach Bedarf insbesondere als Hydrotropikum, Viskositätsregulator und/oder Kältestabilisator eingesetzt. Es wirkt lösungsvermittelnd insbesondere für Tenside und Elektrolyt sowie Parfüm und Farbstoff und trägt so zu deren Einarbeitung bei, verhindert die Ausbildung flüssigkristalliner Phasen und hat Anteil an der Bildung klarer Produkte. Die Viskosität des erfindungsgemäßen Mittels verringert sich mit zunehmender Lösungsmittelmenge. Zuviel Lösungsmittel kann jedoch einen zu starken Viskositätsabfall bewirken. Schließlich sinkt mit zunehmender Lösungsmittelmenge der Kältetrübungs- und Klarpunkt des erfindungsgemäßen Mittels.

Die Komponenten des erfindungsgemäßen Reinigungsmittels können vorteilhafterweise zusätzlich ein oder mehrere Enzyme enthalten, vor allem ausgewählt aus der Gruppe bestehend aus Proteasen, Polysaccharidasen und Nukleasen. Bei der Polysaccharidase kann es sich insbesondere um eine β-Glucanase, Cellulase, Xylanase, Amylase, Dextranase, Glucosidase, Galactosidase, Pectinase, Chitinase, Lysozym und/oder Alginat-Lyase handeln. Bei der Protease kann es sich insbesondere um Subtilisin, Thermolysin, Pepsin, um eine Carboxypeptidase und/oder um eine saure Protease handeln. Bei den Nukleasen kann es sich um jede beliebige DNAse oder RNAse handeln.

Des weiteren kann das erfindungsgemäße Reinigungsmittel gegebenenfalls auch biozide Substanzen enthalten.

Geeignete Lösungsmittel sind beispielsweise gesättigte oder ungesättigte, vorzugsweise gesättigte, verzweigte oder unverzweigte C₁₋₂₀-Kohlenwasserstoffe, bevorzugt C₂₋₁₅-Kohlenwasserstoffe, mit mindestens einer Hydroxygruppe und gegebenenfalls einer oder mehreren Etherfunktionen C-O-C, d.h. die Kohlenstoffatomkette unterbrechenden Sauerstoffatomen. Bevorzugte Lösungsmittel sind jedoch die - gegebenenfalls einseitig mit einem C₁₋₆-Alkanol veretherten - C₂₋₆-Alkylenglykole und Poly-C₂₋₃-alkylenglykolether mit durchschnittlich 1 bis 9 gleichen oder verschiedenen, vorzugsweise gleichen, Alkylenglykolgruppen pro Molekül, z.B. Ethylenglykol, Propylenglykol, Butylenglycol, Diethylenglycol, Dimethoxydiglycol, Dipropylenglycol, Propylenglycolbutylether, Propylenglycolpropylether, Dipropylenglykolmonomethylether sowie PEG. Weitere bevorzugte Lösungsmittel sind die C₁₋₆-Alkohole, wie Methanol, Ethanol, n-Propanol, Isopropanol, n-Butanol, t-Butanol usw., besonders bevorzugt wird Ethanol und/oder Isopropanol eingesetzt.

Als Lösungsvermittler können außer den zuvor beschriebenen Lösungsmitteln beispielsweise auch Alkanolamine sowie Alkylbenzolsulfonate mit 1 bis 3 Kohlenstoffatomen im Alkylrest, z.B. Xylol- oder Cumolsulfonat eingesetzt werden. Weitere einsetzbare Hydrotrope sind z.B. Octylsulfat oder Butylglucosid. Das erfindungsgemäße Mittel kann diese Hydrotrope in Mengen von, bezogen auf die Gesamtzusammensetzung, bis zu 4 Gew.-% enthalten.

In einer Ausführungsform können die erfindungsgemäßen Mittel Abrasivstoffe enthalten. Als Abrasivkomponente können hierbei feste wasserlösliche und wasserunlösliche, vorzugsweise anorganische Verbindungen und Gemische derselben dienen. Hierzu gehören beispielsweise Alkalicarbonate, Alkalibicarbonate und Alkalisulfate, Alkaliborate, Alkaliphosphate, Siliciumdioxid, kristalline oder amorphe Alkalisilikate und Schichtsilikate, feinkristalline Natriumaluminiumsilikate und Calciumcarbonat. Der Vorteil wasserlöslicher Abrasivkomponenten besteht in einer praktisch rückstandsfreien Abspülbarkeit des Mittels. Neben diesen anorganischen Stoffen können auch aus der belebten Natur gewonnene Abrasiva, beispielsweise zerkleinerte Nußschalen oder Hölzer, sowie abriebfeste Kunststoffe, beispielsweise Polyethylenperlen, oder auch Keramik- oder Glaskügelchen Verwendung finden. Das erfindungsgemäße Reinigungsmittel kann Abrasivstoffe in Mengen von bis zu 2 Gew.-% enthalten, bezogen auf die Gesamtzusammensetzung.

Neben den genannten Komponenten können die erfindungsgemäßen Mittel einen oder mehrere weitere Hilfs- und Zusatzstoffe enthalten, wie sie vor allem in Reinigungsmitteln für harte Oberflächen üblich sind. Hierzu zählen insbesondere UV-Stabilisatoren, Korrosionsinhibitoren, Reinigungsverstärker, Antistatika, Konservierungsmittel (z.B. 2-Brom-2-nitropropan-1,3-diol oder eine Isothiazolinon-Bromnitropropandiol-Zubereitung), Parfüm, Farbstoffe, Perlglanzmittel (beispielsweise Glykoldistearat) sowie Trübungsmittel oder auch Hautschutzmittel, wie sie beispielsweise in EP 522 506 beschrieben sind. Die Menge an derartigen Zusätzen liegt üblicherweise nicht über 12 Gew.-% im Reinigungsmittel. Die Untergrenze des Einsatzes hängt von der Art des Zusatzstoffes ab und kann beispielsweise bei Farbstoffen bis zu 0,001 Gew.-% und darunter betragen. Vorzugsweise liegt die Menge an Hilfsstoffen zwischen 0,01 und 7 Gew.-%, insbesondere 0,1 und 4 Gew.-%.

Als Farbstoffe können sämtliche in Haushaltsreinigungsmitteln üblicherweise eingesetzten Farbstoffe verwendet werden. Auch bei den Duftstoffen können sämtliche übliche Parfums zum Einsatz kommen. Bevorzugt sind dabei fruchtige Düfte, beispielsweise Citrus, ferner Pine (Fichte) und Minze sowie blumige Düfte.

Komplexbildner und Bleichmittel, wie etwa Natriumhypochlorit, müssen in dem erfindungsgemäßen Reinigungsmittel nicht enthalten sein, können jedoch gegebenenfalls in geringen Konzentrationen vorhanden sein.

Das Reinigungsmittel kann ein oder mehrere Treibmittel (INCl Propellants), üblicherweise in einer Menge von 1 bis 80 Gew.-%, vorzugsweise 1,5 bis 30 Gew.-%, insbesondere 2 bis 10 Gew.-%, besonders bevorzugt 2,5 bis 8 Gew.-%, äußerst bevorzugt 3 bis 6 Gew.-%, enthalten.

Treibmittel sind erfindungsgemäß üblicherweise Treibgase, insbesondere verflüssigte oder komprimierte Gase. Die Wahl richtet sich nach dem zu versprühenden Produkt und dem Einsatzgebiet. Bei der Verwendung von komprimierten Gasen wie Stickstoff, Kohlendioxid oder Distickstoffoxid, die im allgemeinen in dem flüssigen Reinigungsmittel unlöslich sind, sinkt der Betriebsdruck mit jeder Ventilbetätigung. Im Reinigungsmittel lösliche oder selbst als Lösungsmittel wirkende verflüssigte Gase (Flüssiggase) als Treibmittel bieten den Vorteil gleichbleibenden Betriebsdrucks und gleichmäßiger Verteilung, denn an der Luft verdampft das Treibmittel und nimmt dabei ein mehrhundertfaches Volumen ein.

Geeignet sind demgemäß folgende gemäß INCI bezeichnete Treibmittel: Butane, Carbon Dioxide, Dimethyl Carbonate, Dimethyl Ether, Ethane, Hydrochlorofluorocarbon 22, Hydrochlorofluorocarbon 142b, Hydrofluorocarbon 152a, Hydrofluorocarbon 134a, Hydrofluorocarbon 227ea, Isobutane, Isopentane, Nitrogen, Nitrous Oxide, Pentane, Propane.
Auf Chlorfluorkohlenstoffe (Fluorchlorkohlenwasserstoffe, FCKW) als Treibmittel wird jedoch wegen ihrer schädlichen Wirkung auf den - vor harter UV-Strahlung schützenden - Ozon-Schild der Atmosphäre, die sogenannte Ozon-Schicht, vorzugsweise weitgehend und insbesondere vollständig verzichtet.

Bevorzugte Treibmittel sind Flüssiggase. Flüssiggase sind Gase, die bei meist schon geringen Drücken und 20°C vom gasförmigen in den flüssigen Zustand übergeführt werden können. Insbesondere werden unter Flüssiggasen jedoch die - in Ölraffinerien als Nebenprodukte bei Destillation und Kracken von Erdöl sowie in der Erdgas-Aufbereitung bei der Benzinabscheidung anfallenden - Kohlenwasserstoffe Propan, Propen, Butan, Buten, Isobutan (2-Methylpropan), Isobuten (2-Methylpropen, Isobutylen) und deren Gemische verstanden.

Besonders bevorzugt enthält das Reinigungsmittel als ein oder mehrere Treibmittel Propan, Butan und/oder Isobutan, insbesondere Propan und Butan, äußerst bevorzugt Propan, Butan und Isobutan.

Noch ein weiterer Erfindungsgegenstand ist ein Verfahren zur Reinigung von harten Oberflächen im Haushalt, gegebenenfalls unter Benutzung eines erfindungsgemäßen (Mehrkammer-Systems) Erzeugnisses, bei dem die niedrig-viskosen Lösungen auf die zu reinigende Oberfläche aufgebracht und anschließend, gegebenenfalls nach einer angemessenen Einwirkzeit, mit klarem Wasser abgespült werden. Die hoch-viskose Flüssigkeit kann auch vor dem Abspülen mit einem Tuch, einem Schwamm, einer Bürste oder einem sonstigen zur Reinigung geeigneten Utensil auf Oberfläche verwischt oder verrieben werden, was z.B. bei Anwesenheit von Abrasivstoffen im Reinigungsmittel die Reinigungsleistung verbessert. Schließlich wird die Oberfläche gegebenenfalls mit einem trockenen Tuch abgetrocknet. Bei starker Verschmutzung der Oberfläche kann der Reinigungsvorgang anschließend wiederholt werden.

### Haarpflegemittel

Für die Verwendung als Körperpflegemittel, insbesondere als Haarpflegemittel können weitere Bestandteile, insbesondere ausgewählt aus milden Tensiden, Ölkörpern, Emulgatoren, Überfettungsmitteln, Perlglanzwachsen, Konsistenzgebern, Verdickungsmitteln, Polymeren, Siliconverbindungen, Fetten, Wachsen, Stabilisatoren, biogenen Wirkstoffen, Antischuppenmitteln, Filmbildnern, Quellmitteln, Antioxidantien, Hydrotropen, Konservierungsmitteln, Solubilisatoren, Parfümölen und Farbstoffen enthalten sein.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

Als Emulgatoren kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkyl- und/oder Alkenylmono- und -oligoglycoside mit 8 bis 22 Kohlenstoffatomen im Alk(en)ylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEGalkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 PS als Rückfettungsmittel für kosmetische Zubereitungen bekannt.

Alkyl- und/oder Alkenylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder - Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete Verdickungsmittel, die gegebenenfalls in geringen Mengen vorhanden sein können, sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Januar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, VinylpyrrolidonNinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproyl-methacrylat-Copolymere, Polyvinylpyrrolidon, VinylpyrrolidonNinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976).

Typische Beispiele für Fette sind Glyceride, als Wachse kommen u.a. natürliche Wachse, wie z.B. Candelillawachs, Carnaubawachs, Japanwachs, Espartograswachs, Korkwachs, Guarumawachs, Reis-keimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als Stabilisatoren können Metallsalze von Fettsäuren, wie z.B. Magnesium-, Aluminium- und/oder Zinkstearat bzw. -ricinoleat eingesetzt werden.

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als keimhemmende Mittel, die gegebenenfalls zusätzlich zu den zur Hemmung der Adhäsion von Mikroorganismen eingesetzten Patchouliöl, Patchoulialkohol und/oder dessen Derivate den erfindungsgemäßen Kosmetika zugesetzt werden können, sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Menthol, Minzöl, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Enzyminhibitoren können den erfindungsgemäßen Kosmetika ebenfalls zugesetzt werden. Beispielsweise sind Esteraseinhibitoren möglicherweise geeignete Enzyminhibitoren. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder - phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Antischuppenmittel können Climbazol, Octopirox und Zinkpyrethion eingesetzt werden. Bevorzugt können zur Bekämpfung von Kopfschuppen die erfindungsgemäßen Zubereitungen mit mindestens einem dieser Antischuppenmittel kombiniert eingesetzt werden.

Gebräuchliche Filmbildner sind beispielsweise Chitosan, mikrokristallines Chitosan, quaterniertes Chitosan, Polyvinylpyrrolidon, Vinylpyrrolidon-VinylacetatCopolymerisate, Polymere der Acrylsäurereihe, quaternäre Cellulose-Derivate, Kollagen, Hyaluronsäure bzw. deren Salze und ähnliche Verbindungen.

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108, 95 (1993) entnommen werden.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-toluamid, 1,2-Pentandiol oder Ethyl Butylacetylaminopropionate in Frage, als Selbstbräuner eignet sich Dihydroxyaceton.

Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Vorbehandlungsmittel für Wäsche

Für die Verwendung als Vorbehandlungsmittel für Wäsche können neben dem hydrophob-modifiziertem Polyacrylat und den oben genannten Tensiden weitere Komponenten in dem Wäschevorbehandlungsmittel enthalten sein, die dem Fachmann bekannt sind.
Es kann sich hierbei beispielsweise handeln um hydrophobe Komponenten, Gerüststoffe (Builder), Bleichmittel, Enzyme, Parfums/ Duftstoffe, Farbstoffe oder Emulsionshilfsmittel oder Kombinationen davon.

Als hydrophobe Komponenten eignen sich alle bekannten Öle, Fette und Wachse mineralischer, tierischer, pflanzlicher und synthetischer Herkunft. Bevorzugt sind Öl- und Fettkomponenten, vor allem flüssige Kohlenwasserstoffe mit 10 bis 32 C-Atomen, Fettsäureester mit insgesamt 12 bis 26 C-Atomen und beliebige Gemische davon. Geeignete Kohlenwasserstoffe sind vor allem Paraffine und Isoparaffine wie Isohexadecan oder n-Dodecan, aber auch andere, z.B. Triisobuten,

Als hydrophobe Komponenten eignen sich alle bekannten Öle, Fette und Wachse mineralischer, tierischer, pflanzlicher und synthetischer Herkunft. Bevorzugt sind Öl- und Fettkomponenten, vor allem flüssige Kohlenwasserstoffe mit 10 bis 32 C-Atomen, Fettsäureester mit insgesamt 12 bis 26 C-Atomen und beliebige Gemische davon. Geeignete Kohlenwasserstoffe sind vor allem Paraffine und Isoparaffine wie Isohexadecan oder n-Dodecan, aber auch andere, z.B. Triisobuten, Pentapropylen- oder 1,3-Di-(2-ethylhexyl)-cyclohexan. Geeignete Fettsäureester sind z.B. Stoffe wie Methyloleat, Methylpalmitat, Ethyloleat, Isopropylmyristat, n-Hexyllaurat, n-Butylstearat, Glycerinmonooleat, Glycerinmonostearat und Cetyl-/Stearyl-isononanoat. In einer bevorzugten Ausführungsform werden Isoparaffingemische als Hydrophobkomponente eingesetzt. Hierfür kommen beispielsweise die unter den Handelsnamen Cobersol^{®} VPI oder Cobersol^{®} B 105 (CBR) erhältlichen C₁₆₋₂₀-Isoparaffine oder das C₁₂₋₁₄-Isoparaffingemisch Isopar^{®} M (Exxon Mobil) in Frage.
Weitere Stoffe, die als hydrophobe Komponenten verwendet werden können, sind Dialkylether mit insgesamt 12 - 24 C-Atomen. Bevorzugt geeignete Dialkylether sind vor allem die aliphatischen Dialkylether mit jeweils 6 - 10 C-Atomen pro Alkylgruppe.
Darüber hinaus können alle anderen bekannten Ölkomponenten wie Vaseline, Pflanzenöle, synthetische Triglyceride wie z.B. Glyceryl-tricaprylat, aber auch Fette und Wachse sowie Silikonöle in den erfindungsgemäßen Mikroemulsionen enthalten sein. In einer bevorzugten Ausführungsform der Erfindung werden hydrophobe Komponenten, vorzugsweise Paraffine, in Mengen von 5 bis 70 Gew.-%, besonders bevorzugt 8 bis 52 Gew.-%, jeweils bezogen auf die gesamte Mikroemulsion, eingesetzt.

Werden neben Paraffinölen zusätzlich Glycerinmonoester in Mengen von 0 bis 15 Gew.-%, bevorzugt 1 bis 10 Gew.-% eingesetzt, jeweils bezogen auf die gesamte Mikroemulsion, so kann eine Verdickung der erfindungsgemäßen Mikroemulsion eintreten, die aufgrund der somit erreichten höheren Viskositäten von Vorteil bei der bestimmungsgemäßen Anwendung der Mikroemulsion ist. Solcherart verdickte Mikroemulsionen können Viskositäten von 1000 bis 10000 mPas bei einer Scherrate von 30 s⁻¹ und einer Temperatur von 20°C besitzen. Alle weiteren in Wasch- und Reinigungsmitteln üblicherweise eingesetzten Verdickungsmittel, z.B. organische natürliche Verdickungsmittel (Agar-Agar, Carrageen, Traganth, Gummi arabicum, Alginate, Pektine, Polyosen, Guar-Mehl, Johannisbrotbaumkernmehl, Stärke, Dextrine, Gelatine, Casein), organische abgewandelte Naturstoffe (Carboxymethylcellulose und andere Celluloseether, Hydroxyethyl- und -propylcellulose und dergleichen, Kernmehlether), organische vollsynthetische Verdickungsmittel (Polyacryl- und Polymethacryl-Verbindungen, Vinylpolymere, Polycarbonsäuren, Polyether, Polyimine, Polyamide) und anorganische Verdickungsmittel (Polykieselsäuren, Tonmineralien wie Montmorillonite, Zeolithe, Kieselsäuren) können für die erfindungsgemäße Mikroemulsion ebenfalls verwendet werden. Bevorzugt werden jedoch Glycerinmonoester von Fettsäuren eingesetzt, besonders bevorzugt Glycerinmonooleat, welches z.B. unter dem Handelsnamen Monomuls^{®} 90-018 von der Firma Cognis angeboten wird, Glycerinmonostearat, z.B. das von der Firma Cognis erhältliche Cutina^{®} GMS, und Gemische derselben.

Geeignete Builder sind beispielsweise Citronensäure, Alkalimetallcitrate, -gluconate, -nitrilotriacetate, -carbonate und -bicarbonate, insbesondere Natriumcitrat, -gluconat, und -nitrilotriacetat sowie Natrium- und Kaliumcarbonat und -bicarbonat, sowie deren Mischungen. Hierzu zählen auch die Salze der Glutarsäure, Bernsteinsäure, Adipinsäure, Weinsäure und Benzolhexacarbonsäure sowie Phosphonate und Phosphate, beispielsweise Natriumhexametaphosphat. Die Fleckenvorbehandlungsmittel kann Builder in Mengen, bezogen auf die Zusammensetzung, von 0 bis 30 Gew.-% enthalten.

Als Bleichmittel kommen alle üblicherweise in Wasch- und Reinigungsmitteln eingesetzten Bleichmittel in Betracht. Vorzugsweise wird jedoch Wasserstoffperoxid oder Phthalimidoperoxicapronsäure als Bleichmittel verwendet. Das Wäschevorbehandlungsmittel kann Bleichmittel in Mengen von 0 bis 30 Gew.-% enthalten.

In einer besonderen Ausführungsform der Erfindung enthält die Mikroemulsion ein oder mehrere ggf. stabilisierte Enzyme.

Das erfindungsgemäße Fleckenvorbehandlungsmittel kann eines oder mehrere verschiedene amylolytische Enzyme, insbesondere α-Amylasen enthalten. Beispiele für kommerziell erhältliche Amylasen sind BAN^{®}, Termamyl^{®}, Purastar^{®}, Amylase-LT^{®}, Maxamyl^{®}, Duramyl^{®} und/oder Purafect^{®} OxAm.

Insbesondere an chemisch diversen Anschmutzungen kann es vorteilhaft sein, mehrere verschiedene wasch- und/oder reinigungsaktive Enzyme einzusetzen. Dazu gehören beispielsweise Proteasen, aber auch Lipasen, Cutinasen, Esterasen, Pullulanasen, Cellulasen, Hemicellulasen und/oder Xylanasen, sowie deren Gemische. Besonders bevorzugt sind Proteasen, Lipasen, β-Glucanasen und/oder Cellulasen. Weitere Enzyme erweitern die Reinigunsgleistung entsprechender Mittel um ihre jeweils spezifische enzymatische Leistung. Dazu gehören beispielsweise Oxidoreductasen oder Peroxidasen als Komponenten von enzymatischen Bleichsystemen, zum Beispiel Laccasen (WO 00/39306), β-Glucanasen (WO 99/06515 und WO 99/06516) oder Pektin-lösende Enzyme (WO 00/42145), die insbesondere in Spezialmitteln zum Einsatz kommen.

Beispiele für kommerziell erhältliche Enzyme zum Gebrauch in den erfindungsgemäßen Wäschevorbehandlungsmitteln sind Proteasen wie Subtilisin BPN', Properase^{®}, Alkalische Protease aus Bacillus lentus, Optimase^{®}, Opticlean^{®}, Maxatase^{®}, Maxacal^{®}, Maxapem^{®}, Alcalase^{®}, Esperase^{®}, Savinase^{®}, Durazym^{®}, Everlase^{®} und/oder Purafect^{®}G oder Purafect^{®} OxP und Lipasen wie Lipolase^{®}, Lipomax^{®}, Lumafast^{®} und/oder Lipozym^{®}. Die Proteaseaktivität in derartigen Mitteln kann nach der in Tenside, Bd.7 (1970), S. 125-132 beschriebenen Methode ermittelt werden. Sie wird dementsprechend in PE (Protease-Einheiten) angegeben. Die Proteaseaktivität bevorzugter Mittel kann bis zu 1.500.000 Proteaseeinheiten pro Gramm Zubereitung betragen (PE, bestimmt nach der in Tenside, Bd. 7 (1970), S. 125-132 beschriebenen Methode).

Auch diese gegebenenfalls zusätzlich verwendeten Enzyme können, wie zum Beispiel in der europäischen Patentschrift EP 0 564 476 oder in der internationalen Patentanmeldungen WO 94/23005 beschrieben, an Trägerstoffen adsorbiert und/oder in Hüllsubstanzen eingebettet sein, um sie gegen vorzeitige Inaktivierung zu schützen. Sie sind in Waschmitteln vorzugsweise in Mengen bis zu 10 Gew.-%, insbesondere von 0,2 Gew.-% bis 2 Gew.-%, enthalten, wobei besonders bevorzugt gegen oxidativen Abbau stabilisierte Enzyme, wie zum Beispiel aus den internationalen Patentanmeldungen WO 94/18314 bekannt, eingesetzt werden.

Das Fleckenvorbehandlungsmittel kann Enzyme in Mengen von 0 bis 3 Gew.-% enthalten.

Emulsionshilfsmittel, die in den erfindungsgemäßen Mitteln eingesetzt werden können, stammen beispielsweise aus der Gruppe ein- oder mehrwertige Alkohole, Alkanolamine oder Glycolether, sofern sie im angegebenen Konzentrationsbereich mit Wasser mischbar sind. Vorzugsweise werden die Lösungsmittel ausgewählt aus wasserlöslichen ein- oder mehrwertigen Alkoholen mit 1 bis 8 C-Atomen, z. B. Methanol, Ethanol, n- oder i-Propanol, Butanolen, n-Hexanol, n- Octanol, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propylether, Butoxy-propoxy-propanol (BPP), Dipropylenglykolmonomethyl-, oder - ethylether, Di-isopropylenglykolmonomethyl-, oder -ethylether, Methoxy-, Ethoxy-oder Butoxytriglykol, 1-Butoxyethoxy-2-propanol, 3-Methyl-3-methoxybutanol, Propylen-glykol-t-butylether sowie Mischungen dieser Lösungsmittel. Vorzugsweise werden einwertige Alkohole eingesetzt, besonders bevorzugt Ethanol in Mengen von 0 bis 30 Gew.-%, bevorzugt 0 bis 14.5%, und/oder n-Hexanol/n-Octanol in Mengen von 0-10%, vorzugsweise 0-5%.

Weitere organische Lösungsmittel, welche im Prinzip geeignet sind, sind herkömmliche halogenierte Lösungsmittel, wie sie üblicherweise aus der gewerblichen chemischen Reinigung bekannt sind. Hierzu gehören unter anderen die di- bis tetrachlorierten Derivate von Methan, die di- bis pentachlorierten Derivate von Ethan, die mono- bis trichlorierten Derivate von Cyclohexan sowie Monochlorbenzol. Spezielle Beispiele sind Tetrachlorkohlenstoff, Methylenchlorid, 1,1-Dichlorethan, 1,2-Dichlorethan, 1,1,1-Trichlorethan, 1,1,2-Trichlorethan, Trichlorethen, 1,1,2,2-Tetrachlorethan, Tetrachlorethen, Pentachlorethan, Monochlorcyclohexan, 1,4-Dichlorcyclohexan, Monochlorbenzol und Gemische der obigen. Allerdings sind diese chlorierten Kohlenwasserstoffe für den Gebrauch im Haushalt weniger bevorzugt.

Das Fleckenvorbehandlungsmittel kann weiterhin Farbstoffe enthalten. Geeignet sind alle üblicherweise in Wasch- und Reinigungsmitteln eingesetzten Farbstoffe. Die Farbstoffe können insbesondere in Mengen von 0 bis 0,5 Gew.-% enthalten sein.

Weiterhin können Parfums oder Duftstoffe im erfindungsgemäßen Mittel enthalten sein. Hierbei können alle üblicherweise als solche in Wasch- und Reinigungsmitteln eingesetzten Stoffe verwendet werden, vorzugsweise Parfümöle in Mengen von 0-70 Gew.-%, besonders bevorzugt 0-16 Gew.-%.

Gegenstand der Erfindung ist in einer zweiten Ausführungsform die Verwendung der erfindungsgemäßen Textilvorbehandlungsmittel zur Textilvorbehandlung.

Das erfindungsgemäße Fleckenvorbehandlungsmittel ist besonders gut zur gezielten Vorbehandlung von Flecken auf verschiedenen Textilien geeignet. So können Kleidungsstücke und textile Gegenstände aus Vliesen, Filzen, Geweben oder Gewirken von Natur- und Chemiefasern wie Baumwolle, Wolle, Seide, Leinen, Kunstseiden, z. B. Viskose, Polyamid, Polyacryl, Polyester, Polyvinylchlorid, Elastan und allen weiteren üblicherweise eingesetzten Fasern sowie beliebigen Gemischen derselben an verschmutzten Stellen mit dem erfindungsgemäßen Fleckenvorbehandlungsmittel in Kontakt gebracht werden.

In einer Ausführungsform ist der Gegenstand der Erfindung ein Verfahren zur Entfernung von Flecken auf Textilien unter Verwendung eines erfindungsgemäßen Fleckenvorbehandlungsmittels.

Die Anwendung des Fleckenvorbehandlungsmittels erfolgt, indem es, vorzugsweise mit Hilfe einer der obengenannten Applikationsvorrichtungen, auf den Fleck aufgebracht wird und vorzugsweise 5 bis 15 Minuten einwirkt. Besonders bevorzugt wird das Fleckenvorbehandlungsmittel auf dem Fleck verrieben. Anschließend wird es entweder mit klarem Wasser ausgespült, oder aber das Textilstück wird einem sich anschließenden Textilwaschverfahren zugeführt.

### Ausführungsbeispiele:

Die angegebenen Viskositätswerte wurden den Fließversuchen entnommen. Hierbei bedeutet:
η_{0,1s}⁻¹ - Viskosität bei einer Scherrate von 0,1s⁻¹ in Pas
η₁ₛ-1 - Viskosität bei einer Scherrate von 1s⁻¹ in Pas
η₁₀₀ₛ-1 - Viskosität bei einer Scherrate von 100s⁻¹ in Pas.

Die im folgenden angegebenen Gew.-%-Werte für die Tenside Texapon K12, Texapon NSO und Dehydol LS6 beziehen sich jeweils auf den eingesetzten Tensidgehalt, nicht auf das Gewicht der eingesetzten Produkte als ganzes. Texapon K12 enthält 96 Gew.-%, Texpon NSO 27 Gew.-% und Dehydol LS6 98,2 Gew.-% Tensid.

### Beispiel 1:

### Komponente 1:

2 Gew.-% hydrophob modifiziertes Polyacrylat (Molekulargewicht der Polyacrylsäure: 150 kDa, Modifizierungsgrad: 3 % mit C₁₈)
Rest VE-Wasser
pH 9-10

### Komponente 2:

2 Gew.-% hydrophob modifiziertes Polyacrylat (wie in Komponente 1)
2 Gew.-% SDS (Texapon K12 96 %, Cognis, Deutschland)
Rest VE-Wasser
pH 9-10

Die Komponenten 1 und 2 werden im Verhältnis 9 zu 1 miteinander gemischt. Der resultierende pH-Wert liegt bei 9-10.

| Probe | Zusammensetzung | η_{0,1s}-1 (Pas) | η₁ₛ-1 (Pas) | η₁₀₀ₛ-1 (Pas) |
|---|---|---|---|---|
| Komp. 1 | 2% PA 150 kDa | 0,03 | 0,03 | 0,02 |
| Komp. 2 | 2% PA 150 kDa + 2% SDS | 0,05 | 0,05 | 0,04 |
| Gemisch 9:1 | 2% PA 150 kDa + 0,2% SDS | 199,00 | 82,98 | 0,68 |

### Beispiel 2:

### Komponente 1:

0,5 Gew.-% hydrophob modifiziertes Polyacrylat (Molekulargewicht der Polyacrylsäure: 450 kDa, Modifizierungsgrad 3 % mit C₁₈)
Rest VE-Wasser
pH∼9

### Komponente 2:

0,5 Gew.-% hydrophob modifiziertes Polymer (wie in Komponente 1)
1 Gew.-% SDS (Texapon K12 96 %, Cognis, Deutschland)
Rest VE-Wasser
pH∼9

Die Komponenten 1 und 2 werden im Verhältnis 4 zu 1 miteinander gemischt. Der resultierende pH-Wert liegt bei ∼9.

| Probe | Zusammensetzung | n_{0,1s}-1 (Pas) | η₁ₛ-1 (Pas) | η₁₀₀ₛ-1 (Pas) |
|---|---|---|---|---|
| Komp. 1 | 0,5% PA 450 kDa | 0,31 | 0,23 | 0,08 |
| Komp. 2 | 0,5% PA 450 kDa + 1 % SDS | 25,29 | 4,48 | 0,25 |
| Gemisch 4:1 | 0,5% PA 450 kDa + 0,2% SDS | 254,50 | 37,61 | 0,79 |

### Beispiel 3:

### Komponente 1:

2 Gew.-% Acusol 801 S (Rohm und Haas) (entspricht 0,4 Gew.-% hydrophob modifiziertes Polymer)
Rest VE-Wasser
pH∼9-10

### Komponente 2:

2 Gew.-% Acusol 801 S (Rohm und Haas) (entspricht 0,4 Gew.-% hydrophob modifiziertes Polymer)
1 Gew.-% SDS (Texapon K12 96 %, Cognis, Deutschland)
Rest VE-Wasser
ph∼8

Die Komponenten 1 und 2 werden im Verhältnis 4 zu 1 miteinander gemischt. Der resultierende pH-Wert liegt bei ∼8.

| Probe | Zusammensetzung | η_{0,1s}-1 (Pas) | η₁ₛ-1 (Pas) | η₁₀₀ₛ-1 (Pas) |
|---|---|---|---|---|
| Komp. 1 | 2% Acusol 801 S | 33,78 | 4,27 | 0,13 |
| Komp. 2 | 2% Acusol 801 S + 1 % SDS | 1,90 | 0,82 | 0,05 |
| Gemisch 4:1 | 2% Acusol 801 S + 0,2% SDS | 411,60 | 30,99 | 0,64 |

### Beispiel 4:

### Komponente 1:

3,3 Gew.-% Acusol 820 (Rohm und Haas, Deutschland) (entspricht 1 Gew.-% hydrophob modifiziertes Polymer)
Rest VE-Wasser
pH∼6-8

### Komponente 2:

3,3 Gew.-% Acusol 820 (Rohm und Haas, Deutschland) (entspricht 1 Gew.-% hydrophob modifiziertes Polymer)
10 Gew.-% SDS (Natriumdodecylsulfat; Texapon K12 96 %, Cognis, Deutschland) Rest VE-Wasser
pH∼10

Die Komponenten 1 und 2 werden im Verhältnis 49 zu 1 miteinander gemischt. Der resultierende pH-Wert liegt bei 6-7.

| Probe | Zusammensetzung | η_{0,1s}-1 (Pas) | η₁ₛ-1 (Pas) | η₁₀₀ₛ-1 (Pas) |
|---|---|---|---|---|
| Komp. 1 | 3,3% Acusol 820 | 185,70 | 29,27 | 0,83 |
| Komp. 2 | 3,3% Acusol 820 + 10% SDS | 1,46 | 1,29 | 0,18 |
| Gemisch 49:1 | 3,3% Acusol 820 + 0,2% SDS | 332,20 | 72,92 | 5,13 |

### Beispiel 5:

### Komponente 1:

3,3 Gew.-% Acusol 820 (Rohm und Haas, Deutschland) (entspricht 1 Gew.-% hydrophob modifiziertes Polymer)
Rest VE-Wasser
pH∼6-7

### Komponente 2:

3,3 Gew.-% Acusol 820 (Rohm und Haas, Deutschland) (entspricht 1 Gew.-% hydrophob modifiziertes Polymer)
2 Gew.-% SDS (Natriumdodecylsulfat; Texapon K12 96 %, Cognis, Deutschland)
Rest VE-Wasser
pH∼7

Die Komponenten 1 und 2 werden im Verhältnis 9 zu 1 miteinander gemischt. Der resultierende pH-Wert liegt bei ∼7.

| Probe | Zusammensetzung | η_{0,1s}-1 (Pas) | η₁ₛ-1 (Pas) | η₁₀₀ₛ-1 (Pas) |
|---|---|---|---|---|
| Komp.1 | 3,3% Acusol 820 | 194,30 | 31,70 | 0,72 |
| Komp.2 | 3,3% Acusol 820 + 2% SDS | 76,79 | 36,68 | 0,87 |
| Gemisch 9:1 | 3,3% Acusol 820 + 0,2% SDS | 392,20 | 150,40 | 2,96 |

### Beispiel 6:

### Komponente 1:

2 Gew.-% Acusol 801S (Rohm und Haas, Deutschland) (entspricht 0,4 Gew.-% hydrophob modifiziertes Polymer)
Rest VE-Wasser
pH∼11

### Komponente 2:

2 Gew.-% Acusol 801S (Rohm und Haas, Deutschland) (entspricht 0,4 Gew.-% hydrophob modifiziertes Polymer)
0,5 Gew.-% Texapon NSO (Cognis, Deutschland)
Rest VE-Wasser
pH∼8

Die Komponenten 1 und 2 werden im Verhältnis 4 zu 1 miteinander gemischt. Der resultierende pH-Wert liegt bei ∼9.

| Probe | Zusammensetzung | η_{0,1s}-1 (Pas) | η₁ₛ-1 (Pas) | η₁₀₀ₛ-1 (Pas) |
|---|---|---|---|---|
| Komp.1 | 2% Acusol 801 S | 96,07 | 7,37 | 0,16 |
| Komp.2 | 2% Acusol 801 S + 0,5% Texapon NSO | 25,32 | 6,94 | 0,11 |
| Gemisch 4:1 | 2% Acusol 801 S + 0,1% Texapon NSO | 357,30 | 51,26 | 1,12 |

### Beispiel 7:

### Komponente 1:

2 Gew.-% Acusol 801 S (Rohm und Haas, Deutschland) (entspricht 0,4 Gew.-% hydrophob modifiziertes Polymer)
Rest VE-Wasser
pH∼11

### Komponente 2:

2 Gew.-% Acusol 801 S (Rohm und Haas, Deutschland) (entspricht 0,4 Gew.-% hydrophob modifiziertes Polymer)
1,5 Gew.-% Dehydol LS6 (Cognis, Deutschland)
Rest VE-Wasser
pH∼10

Die Komponenten 1 und 2 werden im Verhältnis 14 zu 1 miteinander gemischt. Der resultierende pH-Wert liegt bei ∼10.

| Probe | Zusammensetzung | η_{0,1s}-1 (Pas) | η₁ₛ-1 (Pas) | η₁₀₀ₛ-1 (Pas) |
|---|---|---|---|---|
| Komp.1 | 2% Acusol 801 S | 56,48 | 5,66 | 0,14 |
| Komp.2 | 2% Acusol 801 S + 1,5% Dehydol LS6 | 16,01 | 10,24 | 1,62 |
| Gemisch 14:1 | 2% Acusol 801 S + 0,1% Dehydol LS6 | 289,70 | 44,41 | 0,84 |

### Beispiel 8:

### Komponente 1:

2 Gew.-% Acusol 801 S (Rohm und Haas, Deutschland) (entspricht 0,4 Gew.-% hydrophob modifiziertes Polymer)
Rest VE-Wasser
pH∼11

### Komponente 2:

2 Gew.-% Acusol 801S (Rohm und Haas, Deutschland) (entspricht 0,4 Gew.-% hydrophob modifiziertes Polymer)
5 Gew.-% Dehydol LS6 (Cognis, Deutschland)
Rest VE-Wasser
pH∼10

Die Komponenten 1 und 2 werden im Verhältnis 49 zu 1 miteinander gemischt. Der resultierende pH-Wert liegt bei ∼10.

| Probe | Zusammensetzung | η_{0,1s}-1 (Pas) | η₁ₛ-1 (Pas) | η₁₀₀ₛ-1 (Pas) |
|---|---|---|---|---|
| Komp.1 | 2% Acusol 801 S | 56,48 | 5,66 | 0,14 |
| Komp.2 | 2% Acusol 801 S + 5% Dehydol LS6 | 6,65 | 3,73 | 1,13 |
| Gemisch 49:1 | 2% Acusol 801 S + 0,1% Dehydol LS6 | 537,20 | 46,35 | 0,99 |

### Beispiel 9:

### Komponente 1:

1,0 - 2,5 Gew.-% Acusol 801 S (Rohm und Haas, Deutschland) (entspricht 0,4 Gew.-% hydrophob modifiziertes Polymer)
2 - 10 Gew.-% Glycerin (99,5%)
2 - 10 Gew.-% Monoethanolamin (99%)
Natronlauge (50%) zur Einstellung des pH-Wertes
Rest VE-Wasser
pH 11-13

### Komponente 2:

1,0 - 2,5 Gew.-% Acusol 801 S (Rohm und Haas, Deutschland) (entspricht 0,4 Gew.-% hydrophob modifiziertes Polymer)
0,5 - 3,0 Gew.-% Texapon LS 35 (∼35%; Cognis, Deutschland)
0,1 - 1,5 Gew.-% Dehydol LS6 (Cognis, Deutschland)
2 - 10 Gew.-% Monoethanolamin (99%)
Natronlauge (50 %) zur Einstellung des pH-Wertes
Rest VE-Wasser
pH 11-13

Die Komponenten 1 und 2 werden im Verhältnis 1 zu 1 miteinander gemischt. Der resultierende pH-Wert liegt bei 11-13.

Die vorliegende Erfindung betrifft ein Mehrkomponenten-System, das beim Vermischen eine hochviskose Flüssigkeit ausbildet sowie die Verwendung dieses Mehrkomponenten-Systems, insbesondere als kosmetisches Mittel oder als Wasch- und/oder Spül- und/oder Reinigungsmittel.

In der Technik besteht allgemein ein Bedarf nach Reinigungsmitteln mit hoher Viskosität, um eine verlängerte Kontaktzeit mit der Oberfläche zu realisieren und dadurch den Reinigungseffekt zu verbessern. Ein Problem bei hochviskosen Flüssigkeiten besteht jedoch darin, dass diese in der Praxis nur schlecht zu handhaben sind. So ist es beispielsweise nicht oder nur schwer möglich hochviskose Reinigungsmittel zu dosieren oder unter Verwendung einer Sprühvorrichtung zu applizieren.

Eine Lösung dieses Problems würde etwa darin bestehen, das hochviskose Reinigungsmittel am Applikationsort selbst zu erzeugen. In diesem Sinne wurden Reinigungsmittel entwickelt, die eine niedrige Viskosität besitzen und erst beim Verdünnen mit Wasser eine höhere Viskosität annehmen. So beschreibt etwa EP0595590 ein Konzentrat, das Aminoxide, ein anionisches Alkyl-Detergens, ein hydrophob-modifiziertes Polymer und einen Verdünner enthält. Bei Zugabe von 96 bis 98 Teilen Wasser bildet dieses Konzentrat eine hochviskose Flüssigkeit aus.

Aufgabe der vorliegenden Erfindung war es daher Alternativen zu finden, die es erlauben, am Applikationsort selbst eine hochviskose Flüssigkeit bereitzustellen. Aufgabe der vorliegenden Erfindung war es hierbei insbesondere, eine besser handhabbare Alternative zu den im Stand der Technik beschriebenen Systemen zur Verfügung zu stellen.

Ferner war es Aufgabe der vorliegenden Erfindung, das Auslaufverhalten des Gemisches im Vergleich zum Stand der Technik zu verbessern und/oder eine bessere Verdickung als bei herkömmlichen Verdickersystemen zu ermöglichen.

Die Veröffentlichungen von Iliopoulos et al. (1991) Langmuir 7, 617-619 und Panmai et al. (1999) Colloids and Surfaces A 147, 3-15 beschreiben Flüssigkeiten, die hydrophob modifizierte Polymere enthalten und bei Zugabe von grenzflächenaktiven Substanzen ein Viskositätsmaximum durchlaufen.

Es wurde nun überraschenderweise gefunden, dass ausgehend von mindestens zwei definierten niedrig-viskosen Flüssigkeiten, von denen mindestens eine ein hydrophob modifiziertes Polyacrylat enthält und von denen mindestens eine andere eine Mischung aus einem hydrophob modifizierten Polyacrylat und Detergentien enthält, beim Vermischen dieser in geeignetem Verhältnis, eine hochviskose Flüssigkeit entsteht, insbesondere eine solche, die den Anforderungen eines Reinigungsmittels mit verlängerter Kontaktzeit in vorteilhafter Weise gerecht wird.

Auf diese Weise wird eine erhöhte Produkthaftung an Oberflächen und dadurch eine längere Einwirkzeit ermöglicht, was neben dem erhöhten Reinigungseffekt auch eine Visualisierung für den Verbraucher mit sich bringt.

Gegenstand der vorliegenden Erfindung ist daher ein Verfahren zur Herstellung einer hochviskosen wässrigen Flüssigkeit, **dadurch gekennzeichnet, dass** mindestens zwei, vorzugsweise niedrigviskose, Flüssigkeiten miteinander gemischt werden, wobei die erste der mindestens zwei Flüssigkeiten mindestens ein hydrophob modifiziertes Polyacrylat enthält und die zweite der mindestens zwei Flüssigkeiten mindestens ein hydrophob modifiziertes Polyacrylat und mindestens ein Alkylsulfat oder Fettalkoholethoxylat in einer Menge zwischen 0,05 und 20 Gew.-% enthält, **dadurch gekennzeichnet, dass** die Menge an hydrophob modifiziertem Polymer in den beiden Flüssigkeiten bis zu 5 Gew.-% beträgt, dass die erste und die zweite Flüssigkeit im Verhältnis von 1:2 bis 100:1 miteinander gemischt werden und dass die hydrophobe Gruppe des hydrophob modifizierten Polyacrylats eine Alkylgruppe umfasst.

Gegenstand der vorliegenden Erfindung ist daher des Weiteren ein Zweikomponenten-System, umfassend
a) eine erste wässrige Flüssigkeit, die mindestens ein hydrophob modifiziertes Polyacrylat enthält,
b) eine zweite wässrige Flüssigkeit, die mindestens ein hydrophob modifiziertes Polyacrylat und mindestens ein Alkylsulfat oder Fettalkoholethoxylat in einer Menge zwischen 0,05 und 20 Gew.-% enthält,
wobei die Menge an hydrophob modifiziertem Polymer in den beiden Flüssigkeiten bis zu 5 Gew.-% beträgt, die hydrophobe Gruppe eine Alkylgruppe umfasst, es sich bei dem Zweikomponenten-System um ein Doppelkammer-System handelt und das Verhältnis zwischen erster und zweiter Flüssigkeit zwischen 1:2 und 100:1 liegt.

Erfindungsgemäß beträgt die Menge an hydrophob-modifiziertem Polymer in den beiden Flüssigkeiten vorzugsweise zwischen 0,1 und 2 Gew.-%.

Die erste wässrige Flüssigkeit des Mehrkomponenten-Systems muss kein Tensid enthalten und enthält in einer bevorzugten Ausführungsform auch kein Tensid. In einer weiteren bevorzugten Ausführungsform enthält die erste Flüssigkeit jedoch geringe Mengen an Tensid, vorzugsweise zwischen 0 und 0,4 Gew.-%, besonders bevorzugt zwischen 0 und 0,1 Gew.-%. Es kommt hierbei jedes denkbare Tensid oder Mischungen davon in Frage, insbesondere ein Tensid, das aus derselben Gruppe ausgewählt ist wie das Tensid der zweiten Flüssigkeit. In einer bevorzugten Ausführungsform sind in erster und zweiter Flüssigkeit die gleichen Tenside enthalten.

Die Menge an Tensid in der zweiten Flüssigkeit beträgt vorzugsweise zwischen 0,1 und 12 %, vor allem zwischen 0,2 und 8 Gew.-%.

### Hydrophob modifiziertes Polyacrylat

Das hydrophob modifizierte Polyacrylat kann jede beliebige Struktur besitzen. So kann es beispielsweise linear, verzweigt, kammartig und/oder sternförmig sein.

Hydrophob modifiziert heißt erfindungsgemäß, dass Alkylgruppen an das Polyacrylat gebunden sind und zwar vorzugsweise an ein oder mehrere funktionelle

## Patentansprüche

1. Zweikomponenten-System, umfassend
a) eine erste wässrige Flüssigkeit, die mindestens ein hydrophob modifiziertes Polyacrylat enthält,
b) eine zweite wässrige Flüssigkeit, die mindestens ein hydrophob modifiziertes Polyacrylat und mindestens ein Alkylsulfat oder Fettalkoholethoxylat in einer Menge zwischen 0,05 und 20 Gew.-% enthält,
wobei die Menge an hydrophob modifiziertem Polymer in den beiden Flüssigkeiten bis zu 5 Ges.-% beträgt, die hydrophobe Gruppe eine Alkylgruppe umfasst, es sich bei dem Zweikomponenten-System um ein Doppelkammer-System handelt und das Verhältnis zwischen erster und zweiter Flüssigkeit zwischen 1:2 und 100:1 liegt.

2. Zweikomponenten-System nach Anspruch 1, **dadurch gekennzeichnet, dass** der Modifizierungsgrad des hydrophob modifizierten Polyacrylats zwischen 1 und 10 % liegt.

3. Zweikomponenten-System nach Anspruch 1 oder 2, **dadurch gekennzeichnet, dass** es sich bei dem Tensid um Dodecylsulfat oder ein Fettalkoholethoxylat mit einem mittleren Ethoxylierungsgrad von weniger als 20 EO handelt.

4. Zweikomponenten-System nach Anspruch 3, **dadurch gekennzeichnet, dass** es sich bei dem Tensid um Dodecylsulfat oder ein Fettalkoholethoxylat umfassend einen C₁₂-C₁₈-Fettalkohol und 6-7 Ethoxylat-Einheiten handelt.

5. Zweikomponenten-System nach einem der Ansprüche 1 bis 4, **dadurch gekennzeichnet, dass** die erste Flüssigkeit zusätzlich ein Tensid enthält.

6. Zweikomponenten-System nach Anspruch 5, **dadurch gekennzeichnet, dass** das Tensid in einer Menge zwischen 0 und 0,4 Gew.-% enthalten ist.

7. Zweikomponenten-System nach Anspruch 1, **dadurch gekennzeichnet, dass** es sich bei dem Doppelkammer-System um eine Doppelkammerflasche oder ein Doppelkammerspray handelt.

8. Verfahren zur Herstellung einer hochviskosen wässrigen Flüssigkeit, **dadurch gekennzeichnet, dass** mindestens zwei Flüssigkeiten miteinander gemischt werden,
wobei die erste der mindestens zwei Flüssigkeiten mindestens ein hydrophob modifiziertes Polyacrylat enthält und die zweite der mindestens zwei Flüssigkeiten mindestens ein hydrophob modifiziertes Polyacrylat und mindestens ein Alkylsulfat oder Fettalkoholethoxylat in einer Menge zwischen 0,05 und 20 Gew.-% enthält, **dadurch gekennzeichnet, dass** die Menge an hydrophob modifiziertem Polymer in den beiden Flüssigkeiten bis zu 5 Gew.-% beträgt, dass die erste und die zweite Flüssigkeit im Verhältnis von 1:2 bis 100:1 miteinander gemischt werden und dass die hydrophobe Gruppe des hydrophob modifizierten Polyacrylat eine Alkylgruppe umfasst.

9. Verfahren nach Anspruch 8, **dadurch gekennzeichnet, dass** die Vermischung mit Hilfe eines Zweikammer-Systems erfolgt.

10. Verwendung eines Zweikomponenten-Systems nach einem der Ansprüche 1 bis 7 als kosmetisches Mittel.

11. Verwendung eines Zweikomponenten-Systems nach einem der Ansprüche 1 bis 7 als Wasch- und/oder Spül- und/oder Reinigungsmittel.

## Claims

1. A two-component system comprising,
a) a first aqueous liquid, which contains at least one hydrophobically modified polyacrylate,
b) a second aqueous liquid, which contains at least one hydrophobically modified polyacrylate and at least one alkyl sulfate or fatty alcohol ethoxylate in an amount between 0.05 and 20% by weight,
wherein the amount of hydrophobically modified polymer in both liquids is up to 5% by weight, the hydrophobic group comprises an alkyl group; as for the two-component system, this is a double chamber system and the ratio between the first and the second liquid is between 1:2 and 100:1.

2. The two-component system according to claim 1, **characterized in that** the modification degree of the hydrophobically modified polyacrylate is between 1 and 10%.

3. The two-component system according to claim 1 or 2, **characterized in that,** as for the surfactant, this is dodecyl sulfate or a fatty alcohol ethoxylate with an average ethoxylation degree less than 20 EO.

4. The two-component system according to claim 3, **characterized in that,** as for the surfactant, this is dodecyl sulfate or a fatty alcohol ethoxylate comprising a C₁₂-C₁₈ fatty alcohol and 6-7 ethoxylate units.

5. The two-component system according to any of claims 1 to 4, **characterized in that** the first liquid additionally contains a surfactant.

6. The two-component system according to claim 5, **characterized in that** the surfactant is contained in an amount between 0 and 0.4% by weight.

7. The two-component system according to claim 1, **characterized in that,** as for the double chamber system, this is a double chamber bottle or a double chamber spray.

8. A method for producing a highly viscous aqueous liquid, **characterized in that** at least two liquids are mixed together wherein the first of said at least two liquids contains at least one hydrophobically modified polyacrylate and the second of said at least two liquids contains at least one hydrophobically modified polyacrylate and at least one alkyl sulfate or fatty alcohol ethoxylate in an amount between 0.05 and 20% by weight, **characterized in that** the amount of hydrophobically modified polymer in both liquids is up to 5% by weight, that the first and the second liquids are mixed together in a ratio from 1:2 to 100:1 and that the hydrophobic group of the hydrophobically modified polyacrylate comprises an alkyl group.

9. The method according to claim 8, **characterized in that** the mixing occurs with the help of a two-chamber system.

10. The use of a two-component system according to any of claims 1 to 7 as a cosmetic agent.

11. The use of a two-component system according to any of claims 1 to 7 as a washing and/or rinsing and/or cleaning agent.

## Revendications

1. Système à deux composants comprenant
a) un premier liquide aqueux qui contient au moins un polyacrylate modifié pour le rendre hydrophobe ;
b) un deuxième liquide aqueux qui contient au moins un polyacrylate modifié pour le rendre hydrophobe et au moins un alkylsulfate ou un éthoxylate d'alcool gras en une quantité entre 0,05 et 20 % en poids ;
dans lequel la quantité du polymère modifié pour le rendre hydrophobe dans les deux liquides s'élève jusqu'à 5 % en poids, le groupe hydrophobe comprend un groupe alkyle, en ce qui concerne le système à deux composants, il s'agit d'un système à chambre double et le rapport entre le premier et le deuxième liquide se situe entre 1:2 et 100:1.

2. Système à deux composants selon la revendication 1, **caractérisé en ce que** le degré de modification du polyacrylate modifié pour le rendre hydrophobe se situe entre 1 et 10 %.

3. Système à deux composants selon la revendication 1 ou 2, **caractérisé en ce que,** en ce qui concerne l'agent tensioactif, il s'agit du dodécylsulfate ou d'un éthoxylate d'alcool gras possédant un degré d'éthoxylation moyen inférieur à 20 OE.

4. Système à deux composants selon la revendication 3, **caractérisé en ce que,** en ce qui concerne l'agent tensioactif, il s'agit du dodécylsulfate ou d'un éthoxylate d'alcool gras comprenant un alcool gras en C₁₂-C₁₅ et 6 à 7 unités d'éthoxylate.

5. Système à deux composants selon l'une quelconque des revendications 1 à 4, **caractérisé en ce que** le premier liquide contient en outre un agent tensioactif.

6. Système à deux composants selon la revendication 5, **caractérisé en ce que** l'agent tensioactif est contenu en une quantité entre 0 et 0,4 % en poids.

7. Système à deux composants selon la revendication 1, **caractérisé en ce que,** en ce qui concerne le système à chambre double, il s'agit d'un flacon à chambre double ou d'un spray à chambre double.

8. Procédé pour la préparation d'un liquide aqueux très visqueux, **caractérisé en ce qu'**on mélange l'un à l'autre au moins deux liquides, le premier desdits au moins deux liquides contenant au moins un polyacrylate modifié pour le rendre hydrophobe et le deuxième desdits au moins deux liquides contenant au moins un polyacrylate modifié pour le rendre hydrophobe et au moins un alkylsulfate ou un éthoxylate d'alcool gras en une quantité entre 0,05 et 20 % en poids, **caractérisé en ce que** la quantité du polymère modifié pour le rendre hydrophobe, dans les deux liquides, s'élève jusqu'à 5 % en poids, **en ce que** le premier et le deuxième liquide sont mélangés l'un à l'autre dans le rapport de 1:2 à 100:1, et **en ce que** le groupe hydrophobe du polyacrylate modifié pour le rendre hydrophobe comprend un groupe alkyle.

9. Procédé selon la revendication 8, **caractérisé en ce que** le mélange a lieu à l'aide d'un système à double chambre.

10. Utilisation d'un système à deux composants selon l'une quelconque des revendications 1 à 7, à titre d'agent cosmétique.

11. Utilisation d'un système à deux composants selon l'une quelconque des revendications 1 à 7, à titre d'agent de lavage et/ou d'agent de rinçage et/ou d'agent de nettoyage.
